# EUROPEAN PATENT APPLICATION

(11) **EP 4 421 116 A1**
(43) Date of publication of application: **28.08.2024**
(21) Application number: 24157746.9
(22) Date of filing: 15.02.2024
(51) Int. Cl.: C08J 11/24, C07C 69/82, C09J 167/02, B01D 15/00, B01D 15/36

(54) **PRODUCTION OF RECYCLED POLYESTERS FROM POLYESTER WASTE THROUGH CHEMICAL RECYCLING METHODOLOGY**

(30) Priority: 22.02.2023 IT 202300003012
(71) Applicant: Plasta Rei S.r.l., 04012 Cisterna di Latina, Latina (IT)
(72) Inventor: ABBRUZZESI, Giuseppe, I-04012 Cisterna di Latina, LATINA (IT); MAZZARELLA, Fabio, I-04012 Cisterna di Latina, LATINA (IT); BORGOMEO, Luca, I-04012 Cisterna di Latina, LATINA (IT); BORGOMEO, Francesco, I-04012 Cisterna di Latina, LATINA (IT)
(74) Representative: Long, Giorgio

(57) **Abstract**

The present invention relates to a method for preparing a polymeric material consisting of polyester recycled by means of chemical recycling technology by glycolysis from waste, purification and subsequent repolymerization, so as to obtain a purified product with chemical-physical-mechanical features equal to virgin plastic material.

In particular, the present invention relates to a method for preparing a polymeric material comprising or consisting of the steps of:
a) Providing raw material in the form of flakes suitable for the glycolysis process obtained through a pre-treatment process of dirty post-consumer plastic of polyesters, and mostly Polyethylene terephthalate (PET) without distinction of colour and form.
b) Chemical depolymerisation reaction by glycolysis so as to obtain a mixture defined "glycolysis product (GP)" based on the intermediate Bis-2-hydroxyethyl terephthalate (BHET) and low-molecular-weight small oligomers.
c) Purification step of the glycolysis product (GP) by using ion exchange resins and adsorbents for the removal of contaminants and colour and subsequent step of recovery of the pigments separated from the plastic and subsequent evaporation and concentration reactions so as to obtain the pure BHET monomer, intermediate of the PET production.
d) Polycondensation reaction of the pure intermediate monomer to obtain the polyester and subsequent granulation of the pellet aimed at solid phase crystallisation.

A pilot plant and an industrial plant for carrying out the aforesaid method are also described.

## Description

### Technical field of the invention

The present invention relates to a method for the chemical recycling and purification of waste polyesters by glycolysis reaction.

More in particular, the present invention relates to a method for preparing a polymeric material consisting of polyethylene terephthalate recycled by means of chemical recycling technology by glycolysis and subsequent polymerization, so as to obtain a purified product with chemical-physical-mechanical features (high intrinsic viscosity and high molecular weight) equal to virgin plastic material.

The intermediate produced by the glycolysis process can be easily repolymerized in a conventional PET production plant since the final reaction step is the same.

### Background art

Polyethylene terephthalate (PET) is one of the most commonly used plastics, identified by symbol "1" according to the Voluntary Plastic Container Coding System suggested by the Society of the Plastics Industry in 1988. It is especially used in the field of bottles for single-use beverages and in the production of fibres.

PET is a polyester produced from two main industrial processes, the first of which is from a di-acid, terephthalic acid (TPA), and a monoethylene glycol (MEG) diol with the intermediate bis 2-hydroxyethyl terephthalate monomer (BHET). The dual functionality of the starting raw materials allows, by condensation thereof, the formation of a polymer chain the final molecular weight of which determines the mechanical features thereof.

The management of the end-of-life of plastic products and in particular of PET and polyesters, is of fundamental importance so as to avoid generating waste which accumulates in landfills and increases the already alarming numbers of plastic pollution.

At the same time, however, in the plastic polymers market, the demand for other polyesters with quality features such as to allow the use thereof in any field, such as the food field (not only for the production of bottles, but also containers with coupled materials), in the automotive field, in the pharmaceutical field continuously increases, as well as the need for such materials to be recycled and increasingly more performing.

In such a sense, plastic waste can actually be a resource if inserted in a circular recovery system for producing new plastic raw materials or for generating energy if recycling is not possible.

To date, the possible scenarios for managing the end-of-life of plastic are: mechanical recycling, chemical recycling, and energy recovery.

Recycling in general is the process which best allows achieving the "End Of Waste" purpose, i.e., the moment when the waste, subjected to specific treatment, ceases to be such, taking qualitative features such that it can be qualified as a new product.

However, the current plastic waste recycling technologies are essentially based on mechanical recycling. In such a process, after being collected and sorted into "homogeneous" polymer families, the waste is extruded and then reused without modifying the polymer structure.

However, the variety of waste collected leads to granules with additives of various origins which then make it impossible to obtain products which are qualitatively similar to the initial ones. The degradation effect given by the reprocessing temperature also depletes the properties of the polymer. Therefore, mechanically recycled plastics are actually used for a final product which is different from the one from which they were originally recovered, i.e., a product which is often of lower "value".

Chemical recycling instead represents a possible and attractive solution by virtue of the possibility of generating a new polymer which can also be reused for applications with high quality requirements (e.g., food contact). In fact, chemical recycling allows the production of new raw materials, chemically modifying the chemical structure of the post-consumer polymer and obtaining a purified product.

The main chemical recycling technologies of polyesters and PET are based on chemical depolymerisation processes by solvolysis (glycolysis, methanolysis and hydrolysis) and pyrolysis, then depolymerisation and conversion processes. The former include the return to basic and intermediate monomers, decomposing the polymer by means of chemical reactions, which intermediates are then reused to obtain new virgin polymers. The second use high temperatures and gasification for producing hydrocarbon fractions.

With regard to the depolymerisation processes by solvolysis, there is still a need to provide a method which is capable of producing final intermediates and polyesters of high purity, limiting waste as much as possible and operating in an ecologically sustainable and economic manner.

### Summary of the invention

In this context, it is the technical task underlying the present invention to provide a polymeric material recycled at a high technological level which allows achieving several joint objectives: recycling polyethylene terephthalate-based plastic materials regardless of the family of origin, the supply chain and the degree of purity of the plastic waste in input, thus reducing the amounts of plastic in the environment; reducing the use of fossil resources and reducing CO₂ emissions, thus meeting increasingly stringent environmental needs; overcoming the limits of mechanical recycling with a competitive process with respect to other chemical recycling technologies and with a lower product cost with respect to the starting virgin material.

The technical task and object outlined above are substantially achieved by a method comprising the technical features set out in one or more of the appended claims, the definitions of which form an integral part of the present description.

Therefore, an object of the present invention is a method for preparing a polymeric material comprising or consisting of the steps of:
a) providing a raw material based on polyester terephthalate in the form of flakes suitable for the glycolysis process, said raw material being preferably obtained by a pre-treatment process of dirty post-consumer plastic of polyester terephthalates and mostly polyethylene terephthalate (PET) without distinction of colour and form;
b) reacting, preferably by means of OPFR reactors, by chemical depolymerisation by glycolysis, the raw material from step a) obtaining a mixture of the glycolysis product based on the intermediate bis-2-hydroxyethyl terephthalate (BHET) and low-molecular-weight small oligomers;
c) purifying the glycolysis product from step b) by using ion exchange resins and adsorbents for the removal of contaminants and colour, obtaining pure BHET monomer;
d) optionally, reacting by polycondensation the pure BHET monomer from step c), obtaining polyethylene terephthalate, and then crystallising the produced polyethylene terephthalate in a solid phase.

A further object of the present invention is a pilot industrial plant for studying and scaling-up the process as outlined above, comprising or consisting of:
i) two or three stirred reactors and an oscillating tubular reactor (OPFR) configured for glycolysis step b) and/or polymerization step d), connected by connecting tubes to each other and to two distillation and condensation systems, in which one consists of a distillation column, condenser and collector, the other consists only of a condenser and collector, said reactors being preferably heated by means of a diathermic oil thermal control unit and said distillation column being provided with condenser, distillate collection system and vacuum pump;
ii) a column system for purification and decolourisation by means of ion exchange resins and decolourising adsorbents, preferably provided with a regeneration system of the resins themselves and cooling of the material in input, said system being configured for purification step c);
iii) a water pelletisation system configured for the granulation of the molten material in output from the reactors and a double screw extruder for mixing and compounding operations on the products.

A further object of the present invention is an industrial plant for carrying out the above process, the plant comprising or consisting of:
A) a series pre-treatment plant of a waste plastic material of polyester terephthalate, configured to obtain a flake of plastic material according to step a) suitable for a glycolysis treatment;
B) at least one loading reactor of the raw materials in flakes and one or more tubular oscillating reactors (Oscillating Plug Flow Reactor, OPFR) configured for the glycolysis reaction step according to step b);
C) a system of columns packed with ion exchange resins and adsorbents configured for the purification step c), said system of columns being preferably equipped with a regeneration system of the resins themselves and cooling of the incoming material;
D) a series of two CSTR reactors for the evaporation and concentration reactions and a stripping column for the recovery of the solvent;
E) optionally, one or more feeding tanks of the purified BHET intermediate obtained from the glycolysis step b);
F) a plurality of OPFR reactors divided in blocks for the polycondensation reaction of BHET according to step d);
G) a granulation system for the extrusion and production of pellets of polyethylene terephthalate and a finishing and crystallisation system to obtain polyethylene terephthalate with the purity and the chemical-physical and mechanical properties of virgin PET.

The present invention is inspired by the fact that chemical recycling allows overcoming the limits of the aforementioned mechanical processes, since:
- it allows higher recovery rates (mechanical recycling has yields around 30-40%, while chemical recycling achieves yields around 80%);
- glycolysis is a perfectly replicable process, as it is based on known and reproducible chemical reactions and exploits relatively mild and manageable reaction conditions;
- the raw materials used, i.e., plastic waste, are easily available and manageable with very simple and not overly forced chemical-physical pre-treatments;
- it can be applied to different types of dirty post-consumer plastic waste of polyester terephthalates and mostly PET (in the form of beverage bottles, but also other types of containers, fibres, fabrics or other objects) without distinction in terms of colour, form and size;
- it allows returning to the pure starting BHET monomer intermediate and then to the original polymer, skipping the esterification step of the conventional virgin PET production process. This allows a considerable saving of virgin raw materials, since more than 90% of the glycol used for depolymerisation is recovered and reused in subsequent cycles, and of energy;
- it allows obtaining a polymer with the same features as a virgin polymer, of high-quality level without loss of the features thereof such as intrinsic viscosity and molecular weight due to degradation processes which occur in mechanical recycling and with an ideal infinite number of cycles;
- glycolysis is a well-known and relatively simple chemical process with respect to other conventional chemical recycling processes of polyesters and PET such as hydrolysis and methanolysis.

Therefore, glycolysis is preferable as it is faster, more efficient and less expensive in terms of energy and waste, since by using glycol recoverable by distillation and leading to the production of the BHET monomer intermediate, it allows avoiding a process step by passing directly to the polymerisation step.

Further features and advantages of the present invention will become more apparent from the indicative and thus non-limiting description of a preferred, but not exclusive embodiment of the invention.

### Brief description of the drawings

Figure 1 depicts a partial exemplary diagram of the pilot plant according to the invention;
Figures 2A and 2B depict simplified block diagrams of the pilot plant according to the invention in two different operating conditions;
Figure 2C represents the simplified block diagram of the pilot plant in a second embodiment;
Figure 2D represents a diagram of the third CSTR reactor of the embodiment of Figure 2C
Figure 3 depicts an exemplary diagram of the operational granulation and pelletisation unit of the pilot plant according to the invention;
Figure 4 depicts a block diagram of the pre-treatment operations of the method of the invention;
Figure 5 depicts a block diagram of the operational units usable for the operations in figure 4;
Figure 6 depicts a block diagram of the industrial plant for implementing the method according to the invention;
Figure 7 depicts side (left), front (centre) and perspective (right) views of an OPFR reactor according to the invention;
Figure 8 depicts a sectional side view of the mechanical filter of the plant in figure 6;
Figure 9 depicts a block diagram of the purification operations of the glycolysis product of the method of the invention;
Figure 10 depicts a diagrammatic view of a concentration reactor;
Figure 11 depicts a diagrammatic view of a stripping column;
Figure 12 depicts a block diagram of the pelletisation/crystallisation operations of the polycondensation product of the method of the invention;
Figure 13 depicts a block diagram of the pelletisation/crystallisation units of the industrial plant for implementing the method according to the invention.

### Detailed description of the invention

Therefore, a first object of the present invention is a method for preparing a polymeric material comprising or consisting of the steps of:
a) providing a raw material of polyethylene terephthalate in the form of flakes suitable for the glycolysis process, said raw material being obtained by a pre-treatment process of dirty post-consumer plastic of polyester terephthalates, mostly polyethylene terephthalate (PET) without distinction of colour and form;
b) reacting, by chemical depolymerisation by glycolysis, the raw material from step a) obtaining a mixture of the glycolysis product based on the intermediate bis-2-hydroxyethyl terephthalate (BHET) and low-molecular-weight small oligomers;
c) purifying the glycolysis product from step b) by using ion exchange resins and adsorbents for the removal of contaminants and colour, obtaining pure BHET monomer;
d) reacting by polycondensation the pure BHET monomer from step c), obtaining polyethylene terephthalate, and then crystallising the produced polyethylene terephthalate in a solid phase.

The raw material of polyethylene terephthalate (PET) can comprise or consist of post-consumer light, coloured, "light blue", opaque bottles, post-consumer multilayer items with layers of PET coupled with barrier polymers for gas or metallic or polyolefins, printed PET layers, PET fibres or films, such as fabrics or polyester terephthalates, such as poly-trimethylene terephthalate, or polybutylene terephthalate, of any form and type available from the collection of wastes or from industrial production wastes.

In certain preferred embodiments, **step a)** comprises or consists of the following step:
al) mechanical-physical pre-treatment of the incoming material through a series of steps of drying, separation of materials of other chemical composition, separation of metal content and reduction in scales giving a plastic material flake.

More in detail, the raw material based on polyester terephthalates, in particular post-consumer PET, mostly PET stored preferably in the form of compressed bales, is conveyed on an "unpacker" for the removal of the wires containing iron and plastic. Once separated from said materials, the raw material based on polyester terephthalate is transported by conveyor belts to a rotating sieve of the "trommel" type for the elimination of coarse materials. The material then passes through a coarse metal removal system, in particular an "eddy current" metal detector, and is then conveyed to a label removal and pre-washing section from which it is transported at a further ferromagnetic metal removal system (e.g., magnetic metal detector).

The material thus processed is sent by conveyor belts to one or more cutting mills, through which the mincing into flakes occurs, which, by means of an auger, are conveyed to a water or salt water or slightly alkaline flotation tank, in which the heavier polyester fraction is separated by density from the fraction of lighter plastic impurities. The separated polyester fraction is sent to the drying and desiccation step for the elimination of all traces of water, to a fine foreign material separator and to a further "eddy current" metal detector for the further removal of fine metal impurities. Finally, preferably, the material thus obtained is separated by colour through an optical wavelength reader so as to separate the coloured flakes, preferably of any colour, from the colourless and transparent flakes.

In certain preferred embodiments, **step b)** comprises or consists of the following steps:
b1) preparing a paste consisting of the mixture of reagents and flakes coming from the pre-treatment step a) for the depolymerisation reaction;
b2) glycolysis reaction to give bis-2-hydroxyethyl terephthalate (BHET) and small amounts of low molecular weight oligomers.

In **step b1),** preferably, the pre-treated material in step a) and excess ethylene glycol (mono ethylene glycol, MEG) are loaded in a stirred reactor and such a mixture is heated to obtain the reagent paste for subsequent depolymerisation.

Preferably, such a preparation is carried out under the following conditions:
- molar ratios MEG:PET between 8:1 and 2:1, preferably between 3:1 and 4:1;
- temperature between 100°C and 200°C, preferably between 140°C and 160°C.

**Step b2)** includes the transesterification of the polyester PET in the presence of an excess of MEG (molar ratio MEG/PET as defined above between 8:1 and 2:1, or about 4:1) and in the presence of a glycolysis catalyst, preferably selected from sodium carbonate, zinc chloride or zinc acetate, preferably with a continuous nitrogen flow to obtain the BHET recovery.

Preferably, the reaction is carried out in a tubular reactor called Oscillating Plug Flow Reactor (OPFR), capable of allowing high performance both in terms of product quality and in terms of process sustainability with regard to time and thus energy consumption.

The reaction of **step b2)** can be described by the following diagram:

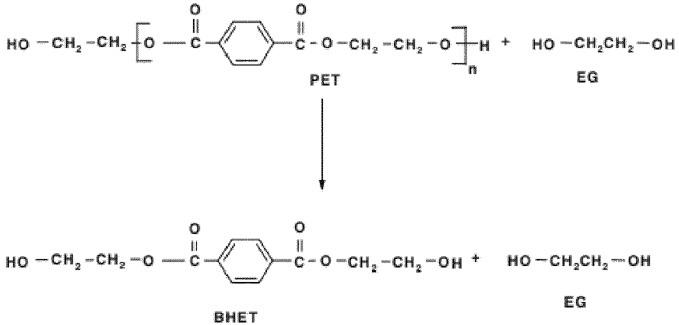

Preferably, the reaction is carried out under the following conditions:
- molar ratios MEG:PET between 8:1 and 2:1, preferably between 3:1 and 4:1;
- catalyst percentage between 0.2-1.5%, preferably about 0.5-1% by weight;
- temperature between 160°C and 260°C, preferably between 180°C and 250°C;
- reaction time between 2 and 8 h, preferably between 4 and 8 h.

Therefore, the reaction of **step b2)** leads to the decomposition of the polyester by reversing the polymerisation reaction with the formation of a mixture of ethylene glycol, the BHET monomer intermediate and low molecular weight oligomers.

The conversion product is then mechanically filtered by a mesh filter to remove any unreacted physical impurities.

In certain preferred embodiments, **step c)** comprises or consists of the following steps:
c1) purification and decolourisation of the glycolysis product with ion exchange resins and adsorbents as a function of the starting raw material (colourless or coloured flakes), with the aim of removing chemical impurities such as metal cations, anions and pigments or colourings and obtaining the purified, clear, and almost colourless BHET monomer;
c2) recovery of the pigments or colourings removed during the decolourisation step;
c3) purification of the glycolysis product by evaporation and concentration suitable to eliminate the excess glycol in order to aid the subsequent polycondensation process.

In **step c1)** the resins used are, respectively, cation exchange, anion exchange and of decolourising adsorbent type for the removal, respectively, of metal ions, anions, and pigments/colourings of the plastic. The resins are packed inside heated columns, in which packed columns the fluid glycolysis product is passed for the time necessary for the adsorption of contaminants.

Once exhausted, the resins are regenerated by counter-current hot washes with ethylene glycol and/or other washing solutions such as solutions of soda, hydrochloric acid and/or sulfuric acid, or acetone.

In embodiments, the resins are selected from:
- cationic matrix resins of polystyrene crosslinked with sulfonic acid functional groups in Na⁺ or H⁺ ionic form.
- anionic resins with styrene matrix with quaternary ammonium functional groups in OH- or Cl-ionic form.
- adsorbent resins with without functional groups.

Preferably **step c1)** is carried out under the following conditions:
- glycolysis product/resin ratio between 10:1 by volume and 1:1 by volume, preferably between 2:1 by volume and 1:1 by volume;
- temperature between 50°C and 180°C, preferably between 70°C and 120°C;

- residence time between 1 and 4 h, preferably between 1 and 2 h.

In **step c2)** the pigments/colourings removed by the adsorbent resin are recovered through solvent washing and distillation. Washing the exhausted resin with an appropriate solvent removes the colour adsorbed thereon, bringing it into solution. The coloured solution is conveyed to the distillation column where the solvent is separated from the pigments which are thus recovered and reusable.

Preferably **step c2)** is carried out under the following conditions:
- resin/solvent ratio between 1:10 and 1:2, preferably between 1:5 and 1:7 by volume;
- washing time between 1 and 4 h, preferably between 1 and 2 h;
- distillation temperature between 40°C and 80°C, preferably between 40°C and 60°C;
- pressure between 1000 and 100 mBar, preferably between 1000 and 500 mBar;
- distillation time between 10 minutes and 1 h, preferably between 10 and 30 minutes.

In **step c3)** the product is subjected to increasing temperatures and decreasing vacuum in two reactors in series: respectively an evaporation reactor and a concentration reactor so as to eliminate more than 90% of the ethylene glycol, which, after being distilled and condensed through the stripping column, is optionally sent to one or more storage tanks to be able to then be reused in the process itself. A recovery of the BHET and oligomers is obtained with a yield between 80 and 99%.

Preferably **step c3)** is carried out under the following conditions:
- temperature between 100°C and 250°C, preferably between 110°C and 140°C;
- pressure between 25 and 500 mBar, preferably between 25 and 200 mBar;
- residence time between 1h and 9h, preferably between 3h and 8h.

The purified BHET can be stored molten, at a temperature between 120°C and 180°C in one or more tanks which will feed the polycondensation section.

In certain embodiments, the BHET obtainable with the methods of steps a), b) and c3) has the features shown in the following table 1.

**Table 1 - Chemical-physical features of the BHET-based glycosylated material.**

| Properties | | Value |
|---|---|---|
| Density | | 1.09-1.14 cm³/g |
| Acid Number | | 0.5-18 mgKOH/g |
| Melting temperature | | 90-115°C |
| Colour | CIE L* | >90 |
| | CIE a* | -2.5/-1.5 |
| | CIE b* | -4/+2 |
| Optical density | | 0.03-0.25 |
| Total cation content | | about 300 ppm |
| Total anion content | | about 700 ppm |
| BHET content | | ≥80% |
| Dimer content | | ≤10% |
| Trimer content | | ≤1% |

In certain preferred embodiments, **step d)** comprises or consists of the following steps:
d1) polycondensation polymerisation reaction of the BHET from step c3) under vacuum in the presence of a Lewis acid catalyst and one acid stabiliser to obtain polyethylene terephthalate (PET), in which said step d1) comprises the recovery of ethylene glycol by distillation;
d2) pelletisation of PET in amorphous phase and solid phase crystallisation of amorphous PET to obtain a crystalline pellet.

**Step d1)** is preferably carried out in the presence of the catalyst in weight percentage of about 0.025% - 0.035%, or about 0.030% and of the stabiliser in weight percentage between 0.0025% and 0.0035%, or about 0.0030%.

The catalyst is preferably antimony oxide (Sb₂O₃), but also titanium oxide (TiO₂), while the stabiliser is preferably phosphoric acid (H₃PO₄).

Other catalysts which can be used are, for example, metal acetates or carboxylates of zinc, magnesium, manganese or cobalt, or compounds with elements of the Fourth Group such as oxides or sulphides.

Other usable stabilisers are for example phosphoric acid esters such as tri-phenyl phosphites and tri-phenyl phosphates.

The section of the polycondensation step consists of the above-mentioned OPFR reactors divided into blocks of one to three or more OPFR reactors. The molten intermediate is preferably fed from a tank. The reaction occurs under vacuum, with different and increasing residual pressures and increasing temperatures in each reactor, so as to ensure a high efficiency of the process in terms of yields, product quality and consumption.

Furthermore, the ethylene glycol contained in the vapours generated during the reaction step is recovered after being conveyed to a stripping column with a thermal gradient of about 80°C (180°C at the bottom and 100°C at the top) : ethylene glycol is recovered at the bottom with a degree of purity such that it can be reused in the process, while volatile substances remain at the top.

Preferably, **step d1)** is carried out with ethylene glycol distillation under the following conditions:
- temperature between 250°C and 295°C, preferably between 280°C and 295°C.
- pressure between residual 30 and 1 mBar, preferably about residual 0.00133 Bar (= 1 mmHg).
- reaction time between about 1 and 9 h, preferably between 2 and 4 h.

The reaction is deemed concluded at a degree of intrinsic viscosity (IV) of 0.60-0.74 (measured according to the ISO 1628-5 or ASTM D 4603 standard) and a Molecular Weight MW (weight average molecular weight) of about 20000-30000.

A reaction diagram of **step d1)** according to a particular form of reaction is shown below:

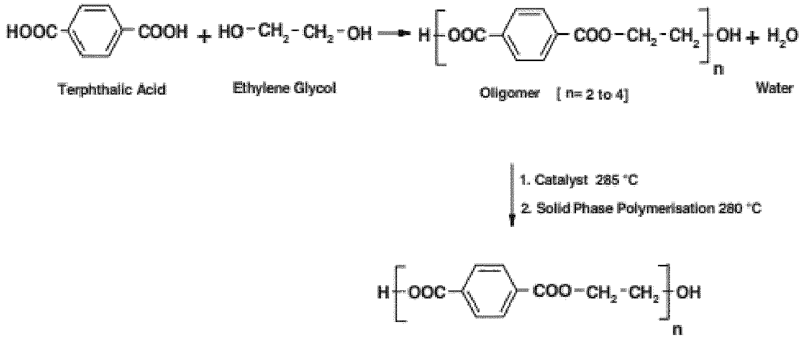

In **step d2),** the pelletisation of the amorphous PET is carried out in a granulation plant which exploits an underwater cutting system. The molten polymer is pushed by a high-pressure pump through holes in a die, at the exit of which it is cut by a series of rotating blades, inside a chamber submerged in a high-speed water flow, which acts as a transport vehicle. The pellets have a size of about 3 mm x 3 mm.

The pellets then undergo a nitrogen flow crystallisation reaction to bring the amorphous PET above the glass transition temperature (Tg) thereof in the crystallisation/finishing plant. The material is conveyed into a first hopper, heated by hot nitrogen at a temperature between 190°C and 240°C, from which the material is sent by pneumatic transport to a second heating step at a lower temperature, between 170°C and 220°C.

The heated polymer undergoes stretching and orientation processes and the polymer chains are rearranged in parallel, becoming tightly packed. Therefore, the crystallisation process consists of nucleation and spherulitic crystallisation.

Preferably, the crystallisation step is carried out under the following conditions:
- temperature between 160°C and 250°C, preferably between 200°C and 240°C;
- reaction time between 12 and 48 h, preferably between 24 and 36 h.

The reaction is considered concluded at a Molecular Weight (weight average molecular weight, MW) >33000 resulting in an intrinsic viscosity value between 0.7-0.9 (measured according to ISO 1628-5 or ASTM D 4603 standard) and a crystallinity percentage greater than or equal to 40%.

Therefore, in certain embodiments, the polymeric material obtainable with the method described above has the features shown in the following table 2.

**Table 2 - Chemical-physical and mechanical features of PET produced with the method of the invention**

| Properties | | Value |
|---|---|---|
| Intrinsic viscosity | | 0.7-0.9 cm³/g |
| Molecular weight (of repetitive units) | | 192 g.mol-1 |
| Average molecular weight MW (weight) | | >33000 g.mol-1 |
| Percentage crystallinity | | 40-50% |
| Density | | 1.3-1.4 g/cm⁻³ |
| Melting density (285°C) | | 1.20 g/cm³ |
| Melt Volume Rate | | 10-30 cm³/10 min |
| Glass transition temperature | | 69-115°C |
| Melting temperature | | 245-265°C |
| Tensile Stress @ Break | | 25-50 MPa |
| Tensile Stress @ Yield | | 25-50 MPa |
| Tensile Modulus | | 1700-2000 MPa |
| Flexural Modulus | | 1000-5000 MPa |
| Flexural Strength | | 50-200 mPa |
| Elongation at break | | 5-15% |
| Deflection temp. at load HDT | | 50-200°C |
| Vicat softening Temp. | | 50-200°C |
| Water absorption (after 24h) | | 0.2-0.7% |
| Moisture content | | ≤0.3% |
| Colour | CIE L* | 77 minimum |
| | CIE a* | -2.5 +/- 1.5 |
| | CIE b* | 0.3 +/- 1.5 |

A second object of the present invention is a pilot industrial plant for studying and scaling-up the process as outlined above, comprising or consisting of the following operational units:
i) two stirred reactors and an oscillating tubular reactor (OPFR) configured for step b) of glycolysis and/or step d) of polymerization, connected by connecting tubes to each other and to two distillation and condensation systems, in which one consists of a distillation column, condenser and collector, the other consists only of a condenser and collector, said reactors being preferably heated by means of a diathermic oil thermal control unit and said distillation column being provided with condenser, distillate collection system and vacuum pump;
ii) a column system for purification and decolourisation by means of ion exchange resins and decolourising adsorbents, preferably provided with a regeneration system of the resins themselves and cooling of the material in input, said system being configured for purification step c);
iii) a water pelletisation system configured for the granulation of the molten material in output from the reactors and a double screw extruder for mixing and compounding operations on the products.

In certain embodiments, the operational units i) and ii) (reactors, column, purification systems) are managed through a computerised system (PLC), while the extrusion and pelletisation system of the operational unit iii) is managed by a specific computerised system.

The reactors are connected to each other and to the purification and granulation system by means of heated and insulated tubes.

In preferred embodiments, **the operational unit i)** comprises or consists of the following equipment:
i1) a first stirred reactor (CSTR A) and a second stirred reactor (CSTR B), preferably in AISI 316/304 steel with a geometric volume of 89 litres, with a jacket for diathermic oil. Preferably, the reactors CSTR A and CSTR B are provided with a stirrer for highly viscous liquids, with motor and inverter, with inlet nozzles for raw materials, nitrogen, steam outlet, distillate return, bottom valve and connecting tubes therebetween and are preferably configured for the following maximum operating temperatures and pressures: shell 300°C/14 bar, jacket 350°C/10 bar;
i2) an oscillating tubular reactor (OPFR), preferably made of AISI 316/304 steel connected to a movement system with a camshaft motor, with a diathermic oil jacket. The OPFR reactor preferably has a geometric volume of about 12 litres and is preferably configured for the following maximum operating temperatures and pressures: shell 300°C/14 bar, jacket 350°C/10 bar;
i3) a distillation column CDI configured for separating water from ethylene glycol and provided with a condenser and condensate collection system with a relaunch pump thereof in the reactors. The column CDI preferably has the following maximum operating temperatures: in operation 190°C; designed 210°C, and the following maximum working pressures: between -1/1 bar; internal maximum pressure 3.5 bar.

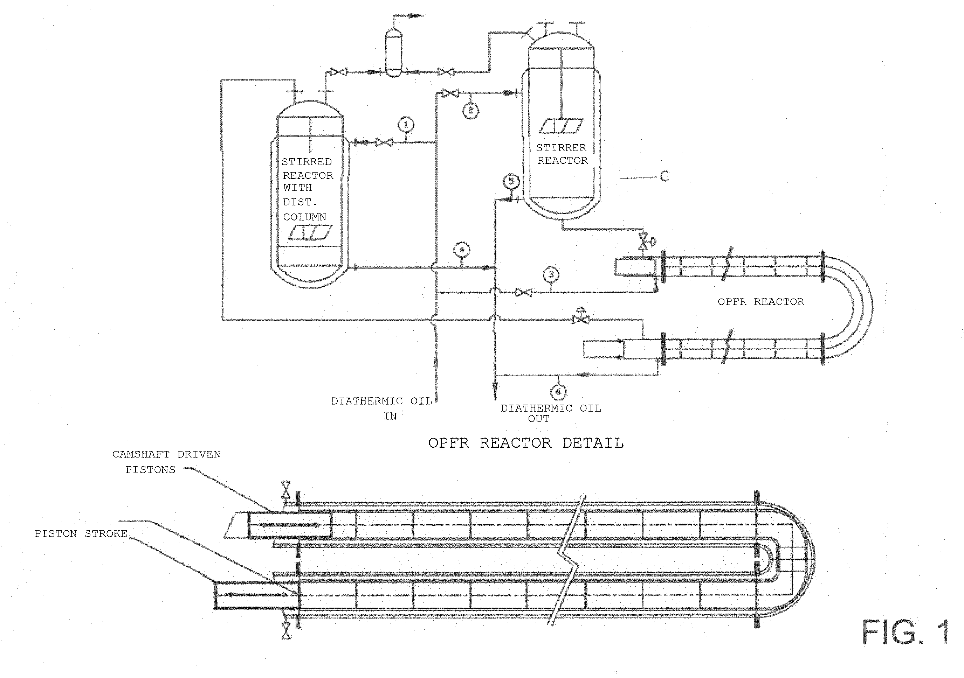

Figure 1 shows a diagram of the equipment i1-i3 according to a particular embodiment.

In other embodiments the operational unit i) includes or is made up of the following equipment:
i1) a first stirred reactor (CSTR A) and a second stirred reactor (CSTR B), preferably in AISI 316/304 steel with a geometric volume of 89 litres, with a jacket for diathermic oil, in which preferably, the CSTR A and CSTR B reactors are equipped with a stirrer for highly viscous liquids, with motor and inverter, with nozzles for raw materials inlet, nitrogen, vapor outlet, distillate return, foot valve and connection pipes between them and are preferably configured for the following maximum temperatures and pressures operating temperature: shell 300°C/14 bar, jacket 350°C/10 bar;
i2) an oscillating tubular reactor (OPFR), preferably made of AISI 316/304 steel connected to a movement system with camshaft motor, with jacket for diathermic oil, in which the OPFR reactor preferably has a geometric volume of approximately 12 liters and is preferably configured for the following maximum operating temperatures and pressures: shell 300°C/14 bar, jacket 350°C/10 bar;
i3) a third stirred reactor (CSTR C) preferably made of AISI 316/304 steel with a geometric volume of 350-500 litres, with jacket for diathermic oil, in which preferably, the CSTR C reactor is equipped with a stirrer for non-viscous liquids, with motor and inverter, with nozzles for raw materials inlet, nitrogen, vapor outlet, condenser and distillate collection system, bottom valve and connection pipes with the OPFR reactor, with the CSTR B reactor and with the operating unit ii) and it is preferably configured for the following maximum operating temperatures and pressures: shell 250°C/10 bar, jacket 250°C/9 bar;
i4) a distillation column CDI configured to separate water from ethylene glycol and equipped with a condenser and condensate collection system with pump for relaunching the same in the reactors, in which the column CDI preferably has the following maximum operating temperatures: operating 190° C; design 210°C, and the following maximum operating pressures: between -1/1 bar; maximum internal pressure 3.5 bar.

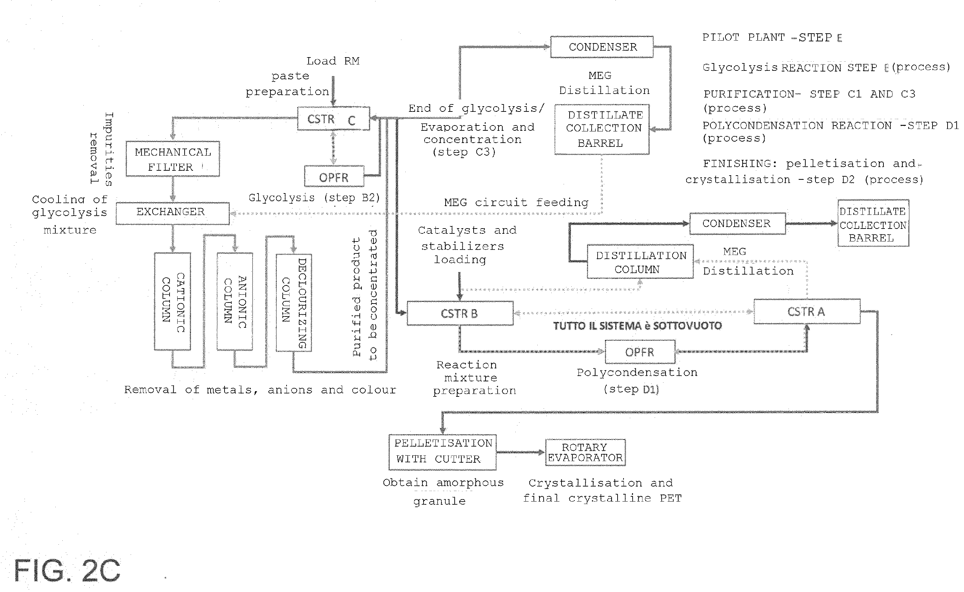

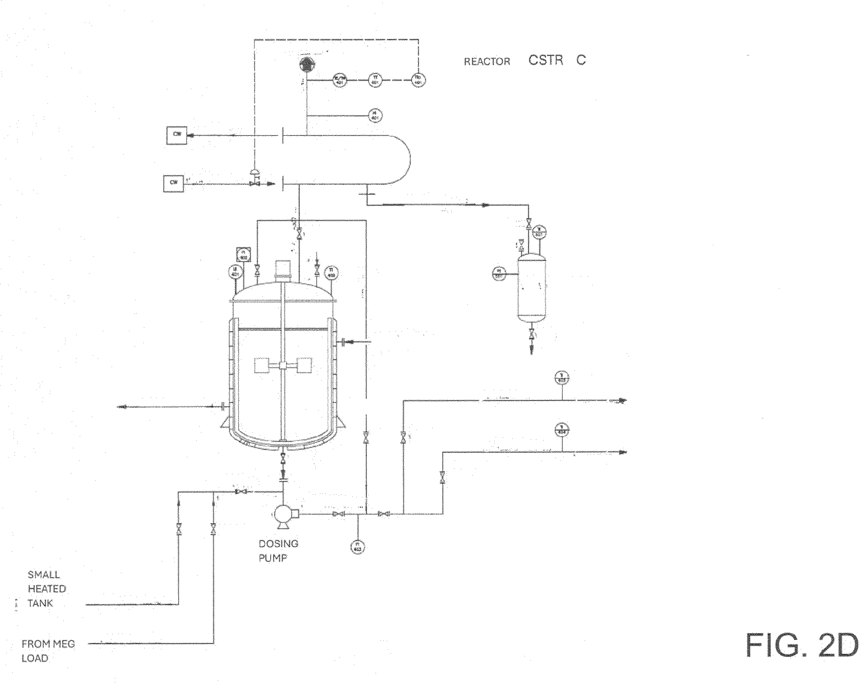

Figure 2C shows a diagram of the pilot plant in accordance with said second embodiment and figure 2D shows a diagram of said third CSTR reactor.

The operational unit ii) comprises or consists of the following equipment:
ii1) mechanical filter M with 100 Mesh filtering mesh to retain impurities undissolved in the reaction step, preferably made with 316 stainless steel tube with 6" diameter, with a height of about 1.5 mt;
ii2) a system of heat exchangers S for cooling the glycolysis product exiting the reactor and entering the equipment ii3), preferably consisting of an ALPHA LAVAL brazed plate heat exchanger mod. CB30-18H and an ALFA LAVAL brazed plate heat exchanger mod. CBH18-23°;
ii3) a plurality of columns placed in series consisting of a first cationic resins column CC, a second anionic resins column CA and a third column CD filled with decolouriser, for the removal of metals, anions, and colour, respectively, by a resin system, preferably made of internal material resistant to the products to be treated such as fibreglass, polypropylene, polyvinylidene fluoride or Teflon. The columns preferably have an internal diameter of about 150 mm, a height of about 1.5 m and a maximum working temperature of 95°C with an attached temperature control and maintenance system and tubes for regenerating the resins themselves.

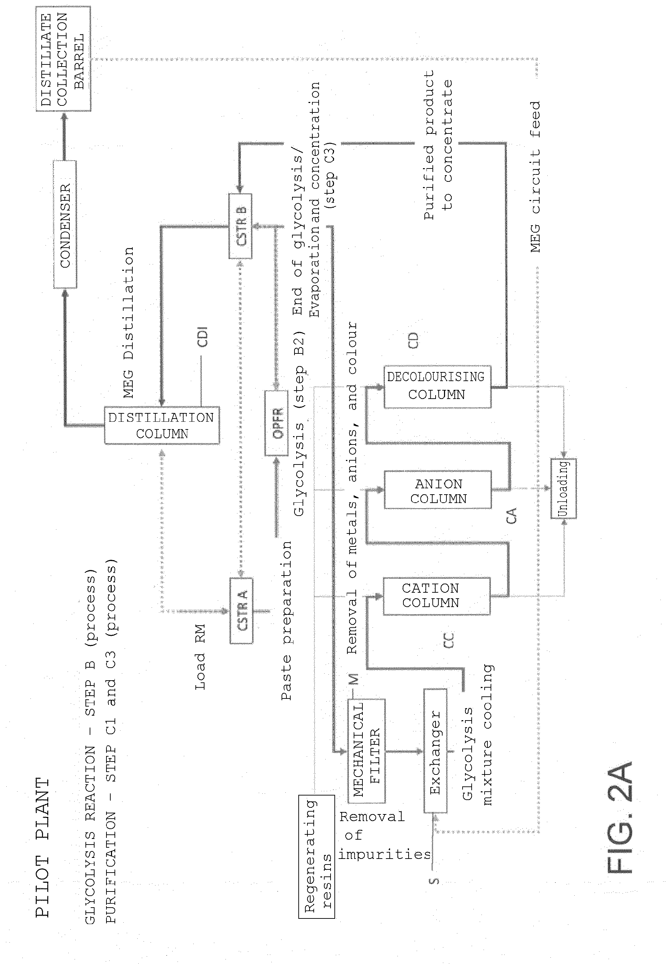

An exemplary diagram of the equipment from ii1-ii3 of the purification step of the pilot plant is shown in figure 2A.

The operational unit iii) comprises or consists of the following equipment:
iii1) double screw extruder E, preferably with screws with a diameter of 16 mm, provided with 3 feeding systems, managed with a computerised system;
iii2) tank and cutter T for the granulation of the molten material usable downhill from the extruder or in an independent manner directly exiting the stirred reactors for the granulation of the molten product.

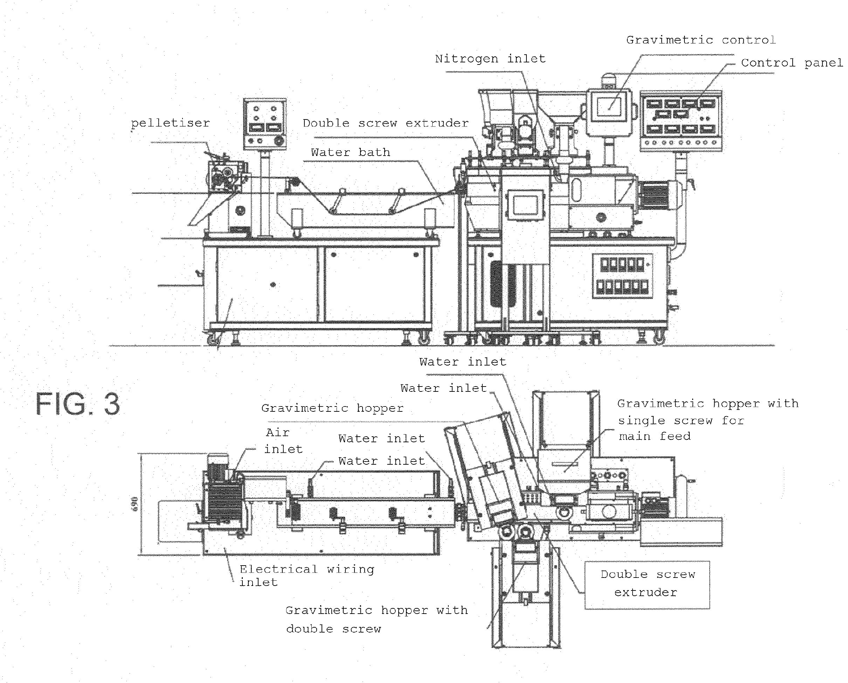

An exemplary diagram of the extrusion and pelletisation system consisting of the equipment iii1-iii2 is shown in figure 3.

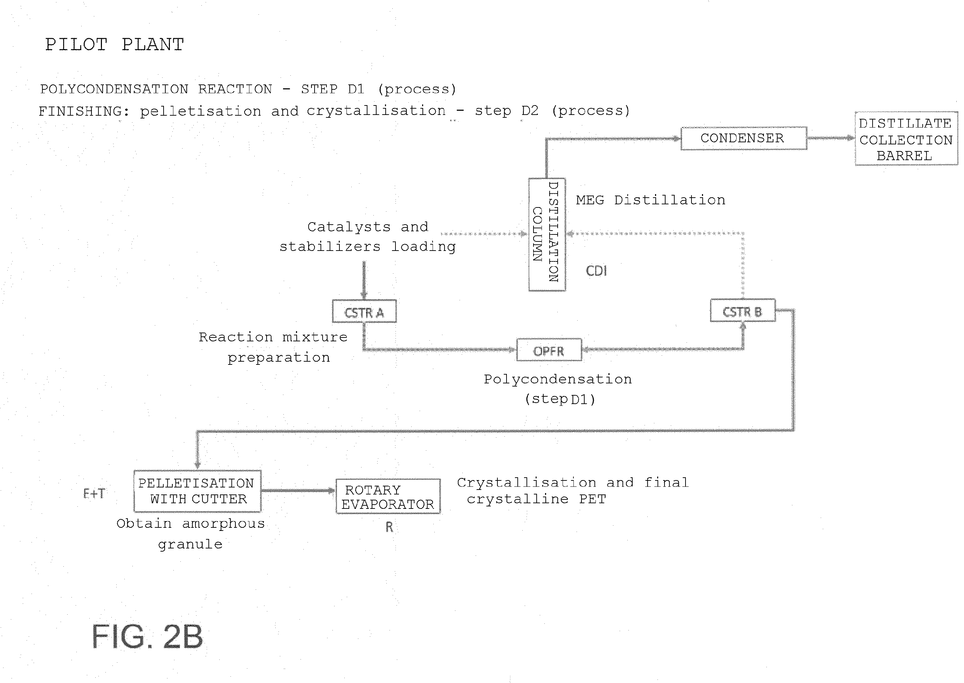

According to the preferred embodiment, as shown in Figures 2A and 2B, by means of the **equipment i1-i3** in a first operating condition (figure 2A) the reactions of **steps b1), b2), c3)** can be carried out, and in a second operating condition (figure 2B) **step d1).**

Steps b1) and b2) are carried out as follows: the raw materials (flakes of polyester, ethylene glycol and catalyst, load RM) are loaded into the first stirred reactor CSTR A, heated to about 150-170°C to promote the dissolution of the flakes in ethylene glycol. When the mass is completely melted, the paste is transferred to the OPFR tubular reactor where the glycolysis reaction occurs (step b2). The reacted mass is collected in the second stirred reactor CSTR B connected to the distillation column C (column-condenser-collection system) of the distillates to always ensure the excess of glycol for the complete conversion of the PET into BHET. In a completely analogous manner, in other embodiments, the raw materials can be loaded into the second stirred reactor CSTR B and the reacted mass can be collected in the first stirred reactor CSTR A which will be connected to the distillation column C (see for example figure 1). The two reactors are thus interchangeable.

Step c3) is carried out as follows: in the second stirred reactor CSTR B in which the purified product is conveyed from the system of the operational unit ii) (see below), the excess ethylene glycol is removed by distillation and recovered through the stripping column connected to the condenser and the condensate collection system.

Step d1) is carried out as follows: the product obtained from step c3) which is in one of the two stirred reactors is added with the catalysts and the stabiliser, brought to the reaction temperature and transferred to the tubular reactor.

During the reaction, by the action of vacuum, ethylene glycol and volatile impurities are removed from the system by the column, condensed and collected in the condensate collector.

The purification and decolourisation of **steps c1) and c2)** is carried out by means of the **equipment ii1-ii3.**

In the resins packed inside the heated columns, the fluid glycolysis product is passed, cooled to the temperature of use of the resins by virtue of the heat exchangers, for the time necessary for the adsorption of the contaminants. Once exhausted, the resins are regenerated by counter-current hot washes with ethylene-glycol and/or other washing solutions such as solutions of soda, hydrochloric acid and/or sulfuric acid, or acetone.

Preferred examples of cationic resins are cationic resins of polystyrene matrix crosslinked with sulfonic acid functional groups in Na⁺ or H⁺ ionic form.

Preferred examples of anionic resins are anionic resins with styrene matrix with quaternary ammonium functional groups in Cl- ionic form.

Preferred examples of adsorbent resins are adsorbent resins with styrene matrix without functional groups.

As shown in figure 2B, the pelletising step of **step d2)** is carried out by means of the **equipment iii1-iii2.**

Step d2) is carried out as follows: once the polycondensation reaction has been completed, the molten polymer is transferred to the granulation step by means of nitrogen pressure through a tube maintained at about 260-270°C and an outlet die with a variable diameter between 2 and 4 mm (equipment E).

The exit of the polymer occurs in the tank-cutter system (equipment T) in water heated to a temperature greater than that of the Tg of the polyester to avoid the vitrification of the molten product. A cooled polymer filament is formed which, through a system of metal guide arms in water, is conveyed to the cutting point where the pellet with dimensions of approximately 3 mm x 3 mm is produced.

The polyester granule could need to be crystallised: for the crystallisation step in the pilot plant, a rotary evaporator R is used, which heats the granule to a certain temperature below 240°C, under vacuum or in a nitrogen stream.

Therefore, the polymeric material obtainable with the pilot plant described above has the features shown in Table 2.

In another embodiment, as shown in figures 2C and 2D, the reactions of phases b1), b2), c3) can be carried out using the equipment i1-i4, and in a second operating condition (as shown in figure 2B) phase d1).

Phases b1) and b2) are carried out as follows: the raw materials (polyester flakes, ethylene glycol and catalyst, load RM) are loaded into the third stirred reactor CSTR C, heated to approximately 150-170°C to favor the dissolution of the flakes in the ethylene glycol. When the mass is completely melted, the paste is transferred to the OPFR tubular reactor where the glycolysis reaction takes place (phase b2). The reacted mass is collected in the same CSTR stirred reactor C connected to the distillate condenser-collector system to always guarantee the excess glycol for the complete conversion of PET into BHET.

Step c3) is carried out as follows: in the CSTR stirred reactor B where the purified product from the operating unit system ii) is conveyed (see below) the excess ethylene glycol is removed by distillation and recovered through the rectification column connected to the condenser and the condensate collection system.

Step d1) is carried out as follows: the product obtained from phase c3) which is found in the CSTR stirred reactor B, into which the purified product from the CSTR C reactor is conveyed, is added with the catalysts and the stabilizer, brought to the reaction temperature and transferred to the tubular reactor.

During the reaction, by the action of the vacuum, ethylene glycol and volatile impurities are removed from the system through the column, condensed and collected in the condensate collector.

The purification and decolourization of phases c1) and c2) is carried out using equipment ii1-ii3.

The fluid glycolysis product, coming from the third CSTR reactor C cooled to the temperature at which the resins are used thanks to the exchangers, is passed through the resins packed inside the heated columns for the time necessary for the adsorption of the contaminants. Once exhausted, the resins are regenerated by hot countercurrent washing with ethylene glycol and/or other washing solutions such as for example soda solutions, hydrochloric and/or sulfuric acid, or acetone.

As shown in figure 2C, the pelletizing phase of phase d2) is carried out using equipment iii1-iii2 .

Step d2) is carried out as follows: once the polycondensation reaction is completed, the molten polymer is transferred to the granulation phase by means of nitrogen pressure via a pipe maintained at approximately 260-270°C and an exit die with a diameter varying between 2 and 4 mm (equipment E).

The exit of the polymer occurs in the tank-cutter system (equipment T) in water heated to a temperature higher than that of the Tg of the polyester to avoid vitrification of the melt. A cooled polymer filament is formed which, through a system of metal guide arms in water, is conveyed to the cutting point where the pellet with dimensions of approximately 3 mm x 3 mm is produced.

The polyester granule may need to be crystallized: for the crystallization phase in the pilot plant a rotary evaporator R is used, which heats the granule to a certain temperature below 240°C, under vacuum or in a stream of nitrogen.

Steps c1), c2) and d2) are performed as previously described in relation to the first embodiment.

The polymeric material obtainable with the pilot plant described above, therefore, has the characteristics shown in Table 2.

A still further object of the present invention is an industrial plant for carrying out the above process, the plant comprising or consisting of the following operational units:
A) a series pre-treatment plant of a waste plastic material of polyester terephthalate, configured to obtain a flake of plastic material according to step a) suitable for a glycolysis treatment;
B) at least one loading reactor of the raw materials in flakes and one or more tubular oscillating reactors (Oscillating Plug Flow Reactor, OPFR) configured for the glycolysis reaction step according to step b);
C) a mechanical filtration system with columns packed with ion exchange resins and adsorbents configured for the purification step c), said system of columns being preferably provided with a regeneration system of the resins themselves and cooling of the incoming material;
D) a series of two CSTR reactors for the evaporation and concentration reactions and a stripping column for the recovery of the solvent;
E) optionally, one or more feeding tanks of the purified BHET intermediate obtained from the glycolysis step b);
F) a plurality of OPFR reactors divided in blocks for the polycondensation reaction of BHET according to step d);
G) a granulation system for the extrusion and production of pellets of polyethylene terephthalate and a finishing and crystallisation system to obtain polyethylene terephthalate with the purity and the chemical-physical and mechanical properties of virgin PET.

In a particular embodiment, the **operational unit A** comprises or consists of the equipment described as follows:
A1) optionally, a metal chain conveyor configured to convey the polyester bales to the unbaling machine;
A2) optionally, an unbaling machine configured for manually cutting the steel packaging line of the packed bottles that will be then opened by a screw blade;
A3) a conveyor for conveying the material to the operational unit downhill, the conveyor being preferably a double screw loader configured for better separating the bottles;
A4) a roller screening system configured for separating impurities such as sand, rocks, metals, caps, based on the different sizes of the holes;
A5) an "eddy *current"* separator configured for separating non-magnetic metal fractions;
A6) a conveyor, preferably a belt one, configured for conveying material to the subsequent step;
A7) a label removal system configured for removing the labels by friction;
A8) a conveyor to convey the material to the operational unit downhill, the conveyor preferably consisting of a horizontal collection belt and a conveyor belt, configured to collect the bottles together from the label removal set and to transport the material to the subsequent step, respectively;
A9) a pre-washing apparatus for the removal of the labels in water, configured for removing labels and sand, earth and other impurities;
A10) a "metal detector" with "pneumatic turnover", configured for removing magnetic metals;
A11) a conveyor, preferably a belt one, configured for transporting material to the operational unit downhill;
A12) a milling system configured for cutting the material in small sizes;
A13) a conveyor configured for conveying the material flakes to the operational unit downhill, the conveyor preferably consisting of horizontal and oblique conveying screws;
A14) at least one floating basin, configured for gravity separating different minced materials from the polyester;
A15) a conveyor, preferably a loading screw, configured for conveying the materials out from the floating basin;
A16) a de-washing machine, configured for reducing the water content exiting the floating basin;
A17) a drying system, preferably a thermal tubing one, configured for reducing the humidity content and dry the material flakes by warm air;
A18) optionally, a zig-zag blower for label removal, configured for separating further small amounts of labels and dust from the material flakes;
A19) a second *"eddy current"* separator, configured for further separating non-magnetic metal fractions;
A20) a colour separator configured for separating flakes of different colours depending on the wavelength and for separating different types of plastics the ones from the others by infrared;
A21) optionally, one or more bins configured for temporary storing polyester flakes.

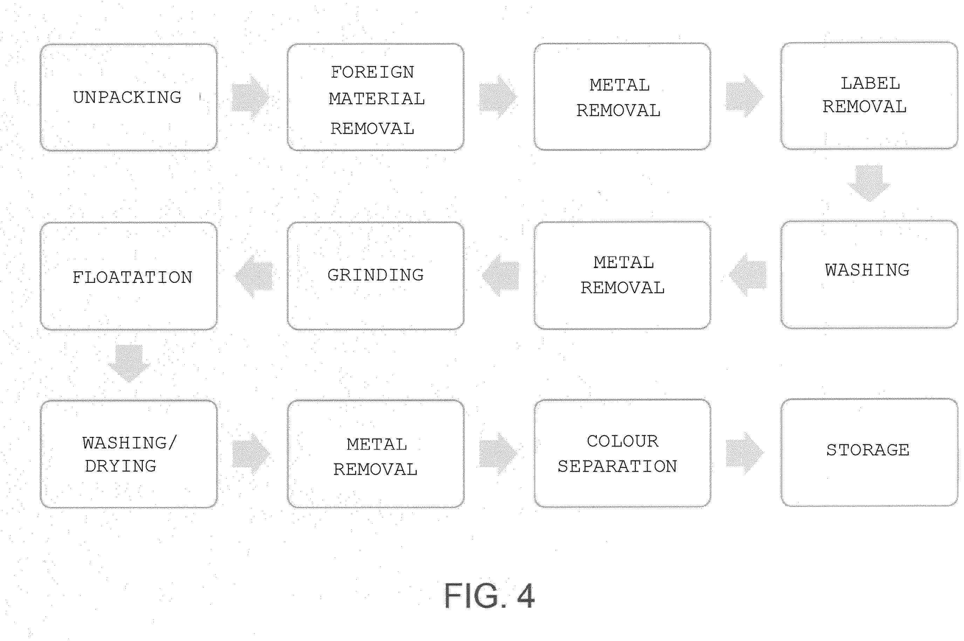

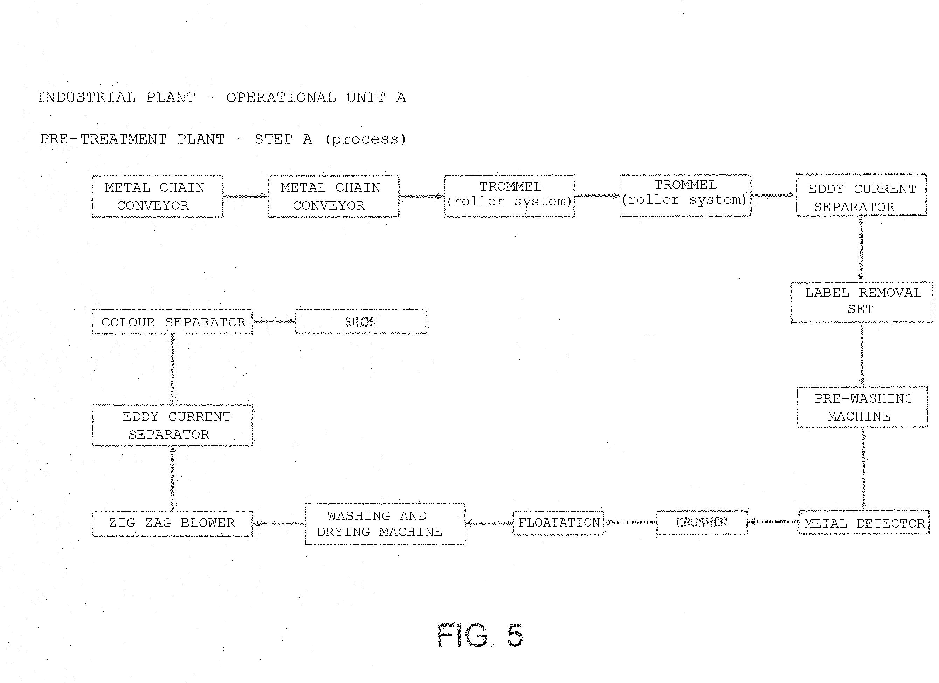

A summary diagram of the operations which can be carried out with the operational units A1-A21 is shown in figure 4 and a block diagram of the operational units A1-A21 is shown in figure 5.

The pre-treatment and preparation of the flakes according to **step a)** of the present invention is carried out by means of the **equipment A1-A21.**

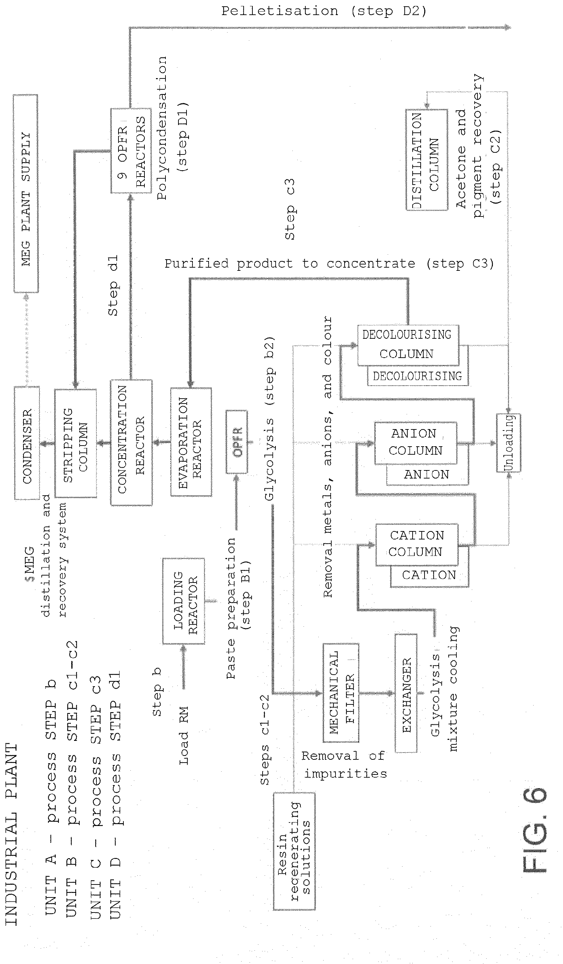

A block diagram of the industrial plant comprising the operational units B to F is shown in figure 6.

In a particular embodiment, the reactors **B** are of two different types, with different construction and operating principles and comprise or consist of:
B1) at least one loading reactor, preferably at least one CE316 steel reactor, with a capacity of 50 m³, suitable for processing up to 205°C capable of working under pressure and vacuum, configured for preparing the glycolysis mixture;
B2) at least one OPFR (Oscillating Plug Flow Reactor) reactor comprising a tubular reactor, preferably of length between 10 and 100 mt, preferably mounted on a skid consisting of stirred and heated barrels, preferably in CE304 or CE316 steel, configured to work under negative pressure conditions and equipped with a transfer pump.

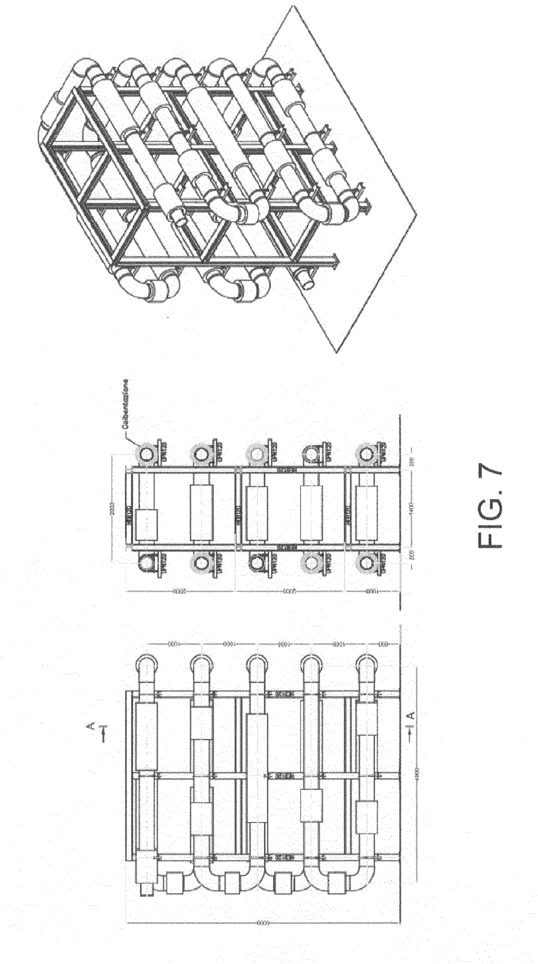

A diagram of the OPFR reactor is shown in figure 7.

The reactions of **steps b1) and b2)** are carried out by means of the reactors of **operational unit B.**

In a particular embodiment, the **operational unit C** comprises or consists of:
C1) a mechanical filter with a filtering mesh from 100 to 1000 microns inside to retain impurities undissolved in the reaction step, preferably of the jacketed basket type and made of AISI316 stainless steel with a diameter of about 220 mm and a height of about 850 mm, resistant to about 6 Bar and 200°C;
C2) a cooling system for the incoming material in the C3 columns, preferably consisting of a buffer reactor for loading the glycolysis product exiting the reactors B) which feeds a LAVAL ALFA brazed plate exchanger for cooling the incoming product in the resins;
C3) a plurality of columns with a resin system for the removal of metals, anions, and colour respectively, positioned in series and divided by type, preferably into three two-column groups with a maximum working temperature of 95°C. The columns are preferably jacketed with hot water, made with internal material resistant to the products to be treated, such as fibreglass, polypropylene, polyvinylidene fluoride or Teflon and have a diameter of about 0.5 m and a height of about 2.5 m. The columns C3) are preferably connected to a tubing system for the regeneration of the resins themselves, including a distillation column for pure acetone used in washing the resin decolouriser and consequent recovery of the colour pigments as a precipitate.

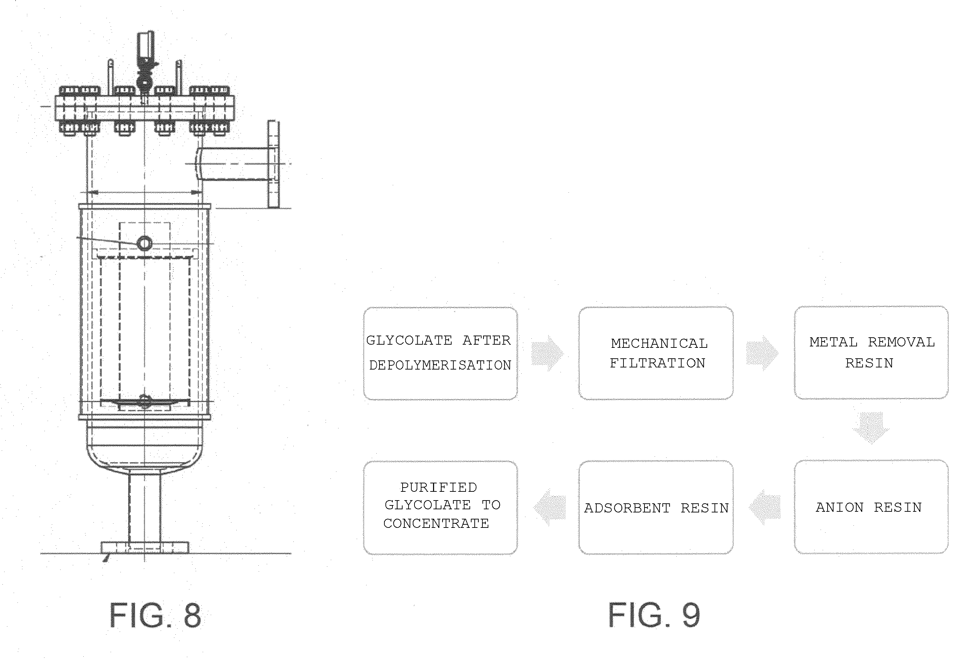

A diagram of the operational units consisting of the equipment C1) to C3) is shown in figure 9, while a sectional side view of the filtration unit C2 is shown in figure 8.

The purification and decolourisation of **steps c1) to c2)** of the method of the invention is carried out by means of **operational units C1-C3.**

In a particular embodiment, the **operational unit D** comprises or consists of:
D1) an evaporation reactor for the first evaporation step of the ethylene glycol solvent from the glycolysis product, said reactor preferably being a CSTR reactor in CE316 and CE304 steel, with a capacity of 25 and 13 m³, configured for pressures up to 10 bar with vacuum system and temperatures up to 350°C;
D2) a concentration reactor for the final elimination step of the ethylene glycol solvent and obtaining BHET with purity between 80% and 99%, said reactor preferably being a CSTR reactor in CE316 and CE304 steel, with a capacity of 25 and 13 m³, configured for pressures up to 10 bar with vacuum system and temperatures up to 350°C;
D3) a stripping column for the condensation and recovery of pure ethylene glycol to be recycled in the process.

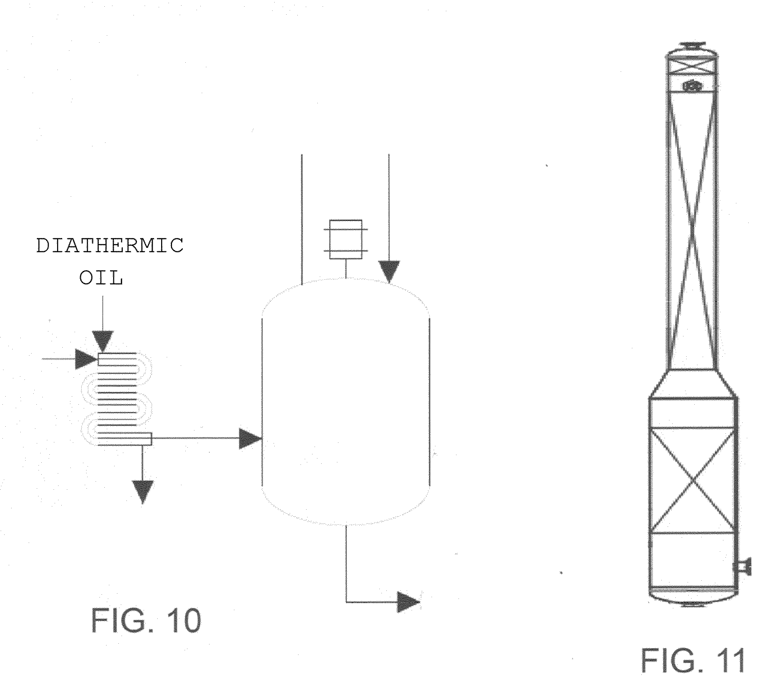

A diagram of a reactor D1 or D2 and column D3 are shown in figures 10 and 11, respectively.

The evaporation and concentration steps of **step c3)** of the method of the invention are carried out by means of the **operational units D1-D3.**

In a particular embodiment, the **operational units F** are of the same type as the operational unit C2 and, in a particular embodiment of the invention, comprise or consist of:
F1) a plurality of tubular OPFR (Oscillating Plug Flow Reactor) reactors, preferably from three to nine OPFR reactors, preferably of lengths between 10 and 100 m, preferably mounted on skids consisting of stirred and heated barrels, and preferably made of CE304 or CE316 steel, designed to work under negative pressure conditions and equipped with a transfer pump.

The reactions of **step d1)** of the method of the invention are carried out by means of the **operational unit F1.**

The **operational unit G** consists of the submerged extrusion and pelletisation system for making the polyester pellets produced in step d1) and of the crystallisation and finishing system of the final product. In a particular embodiment, the operational unit G comprises or consists of:
G1) optionally, a deviating valve, preferably in AV 65 polymer, used to deviate the flow of molten material from the deviation position to the production (matrix) position in a short time. The deviating valve is used to purge the cutting head from deteriorated material before starting the pelletisation system;
G2) an immersion pelletisation unit with a movable support, preferably provided with fully automatic starting of the motor of the pelletiser after closing the cutting chamber. In said unit G2, the molten polymer flows through a matrix having a series of holes. When the polymer exits from the holes, it is cut into granules by rotating blades, then it is solidified when it is washed away by the process water flow passing through the cutting chamber in the centrifugal dryer;
G3) a "Die Plate" unit to ensure the thermal insulation that prevents freezing of the material at the matrix nozzle. The matrix is heated by heater cartridges and the temperature of the component is preferably monitored by a temperature sensor positioned on the body of the support. Preferably, the die is freely accessible if the pelletiser housing is mounted on a mobile unit and can thus be easily cleaned;
G4) a cutting chamber configured for the transport of the cut pellets by the process water. Such an operation is obtained by means of the safety lock with automatic hydraulic closure on the housing of the stainless-steel pelletiser. There are preferably two stainless steel knife holders with hardened steel tool blades. The adjustment of the knife by means of servomotor is preferably controlled automatically, this allows automatically resharpening the knives during operation to ensure consistent pellet quality. Preferably, the electronic monitoring of the pelletisation blades indicates the current wear of the blades and informs the operating staff of the blade change necessary to ensure a safe pelletisation process. The speed range of the blade shaft is preferably controlled by a frequency converter and selected on the operator panel;
G5) optionally, a supporting frame with small wheels on which the whole pelletiser is mounted, to compensate for the thermal expansion of the upstream components. Furthermore, the pelletiser can be moved intuitively for the cleaning and maintenance of the extruder;
G6) a water and drying treatment system including a centrifugal dryer, to which the cut granules are conveyed via the process water pump and separated from the process water. Preferably, the water separator/centrifugal dryer provides maximum residual pellet moisture of about 0.05-0.5% depending on the material properties;
G7) optionally, a water tank configured for the required process water, including a blade sieve with grooved sieve filter. The water is preferably heated by means of flanged electrical resistors and the level is preferably controlled by means of a solenoid valve and a float switch;
G8) optionally, a flow meter for the process water between the process water pump and the pelletisation, displaying the actual flow rate in the control unit of the immersion pelletiser;
G9) a centrifugal dryer/dryer preferably having a large door for easy cleaning, a small door for maintenance and three physically accessible filters. The dryer door and the maintenance door are preferably protected by a safety switch to prevent them from opening during operation;
G10) a discharge fan configured for extracting the water vapour, generating a counterflow to the flow of material from the granulate, to reach the target values of residual humidity;
G11) optionally, an electrical system with PLC in which all the electric components required for control by the operator and monitoring of the machine are installed in the electric board. The PLC provides for the control of the pelletisation line, and the operator panel is set on a rotating arm situated directly at the pelletisation unit. The adjustment of the set point includes all the set points (partly optional) such as temperatures, speeds, parameters, and times set on the operator panel. Furthermore, all the actual values, such as the feed pressure and the speed of the pelletiser, are also displayed on the panel itself;
G12) a solid-state warm crystallisation system equipped with vibrating conveyor, preferably driven by two unbalanced motors (0.3 kW each), which keeps the pellet initially still amorphous under continuous movement, so as to prevent a conglutination of the pellets. Due to the specific heat of the pellets and the low packing density, a high concentration of thermal energy develops and the pellets are transformed into a semi-crystalline state.

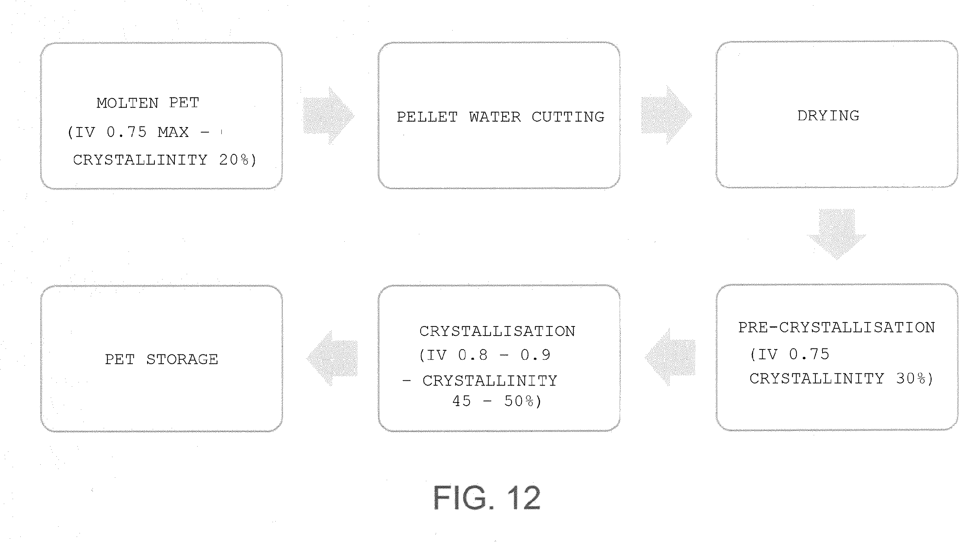

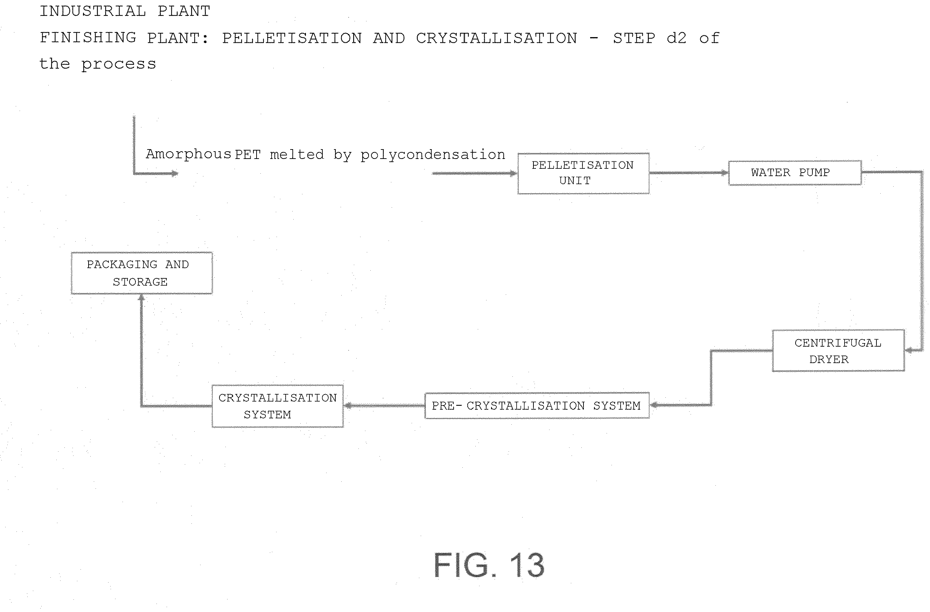

An exemplary diagram of the operations implemented with the operational units G1-G12 is shown in figure 12, while an exemplary diagram of the operational units G1-G12 is shown in figure 13.

The pelletisation and crystallisation of **step d2)** is carried out by means of the **operational units** G1-G12.

### Specific examples shown in the figures

### Figure 1

The following operating parameters are used in operations 1 to 6:

| Operation | Flow rate (m³/hr) | Pressure (bar) | Temperature (°C) |
|---|---|---|---|
| 1) | 15 | 5 | 100-310 |
| 2) | 15 | 5 | 100-310 |

| | | | |
|---|---|---|---|
| 3) | 15 | 5 | 100-310 |
| 4) | 15 | 5 | 95-305 |
| 5) | 15 | 5 | 95-305 |
| 6) | 15 | 5 | 95-305 |

The OPFR reactor is operated with a piston speed of 113 mm/min and a pressure of 3 bar FV.

### Figure 5

In the order indicated by the arrows starting from the block in the upper left:
METAL CHAIN CONVEYOR: Post-consumer plastic bale transport system
METAL CHAIN CONVEYOR: Unpacking the post-consumer plastic
TROMMEL (roller system): separating coarse impurities
METAL CHAIN CONVEYOR: unpacking post-consumer plastic
EDDY CURRENT SEPARATOR: separating non-magnetic metal fractions
LABEL REMOVAL SET: Removing labels by friction
PRE-WASHING MACHINE: removing labels in water and removing impurities
METAL DETECTOR: removing magnetic metal
CRUSHER: cutting the material into small sizes
FLOATATION: separating extraneous plastics from Polyester by gravity
WASHING AND DRYING MACHINE: reducing the water content and drying the flakes
ZIGZAG BLOWER: separating labels and dust from the flakes
EDDY CURRENT SEPARATOR: separating non-magnetic metal fractions
COLOUR SEPARATOR: separating flakes based on type of plastic and colour
SILOS: temporary storage of polyester flakes.

### Figure 13

In the order indicated by the arrows starting from the block in the upper left:
Polycondensation amorphous molten PET: IV max 0.75, crystallinity about 200
PELLETISATION UNIT: the molten polymer flows through a series of holes and is cut into granules by rotating blades in water
WATER PUMP: the pellet is conveyed by transport on water to the drying step
CENTRIFUGAL DRYER: the process water is removed from the pellet, ensuring residual humidity of about 0.05-0.5%
PRE-CRYSTALLISATION SYSTEM: IV 0.75, Crystallinity about 30%
CRYSTALLISATION SYSTEM: IV 0.8-0.9, crystallinity about 45-50%; vibration conveyor which keeps the still amorphous pellet in continuous movement, preventing pellet conglutination. A high concentration of thermal energy is developed and the pellets are transformed into a semi-crystalline state.

The advantages of the present method and the plant for the implementation thereof are evident from the above.

In fact, although the glycolysis of PET is in itself a known process, the method of the invention stands out in that it allows improving the final features of the PET produced and the recovery of the materials used or present in the waste plastic material used (including coloured pigments).

Therefore, the technological innovation represented is evident in the fact that the process allows recovering a waste practically in the entirety thereof, avoiding excessive pretreatments, with the aim of using it without distinction in terms of colour, form, and type by virtue of the purification technologies of the invention.

The polyester PET thus obtained is a new pure raw material identical to the virgin production material, produced with a process developed in several steps all within a single industrial plant and which can be marketed as such or mixed with any other plastic components.

It is apparent that only some particular embodiments of the present invention have been described, to which those skilled in the art will be able to make all changes required to adapt it to particular applications, without departing from the scope of protection of the present invention.

## Claims

1. A method for preparing a polymeric material including or consisting of the steps:
a) providing a raw material of polyethylene terephthalate in the form of flakes suitable for the glycolysis process, said raw material being preferably obtained by a pre-treatment process of dirty post-consumer plastic of polyester terephthalates, in particular, polyethylene terephthalate (PET) without distinction of colour and form;
b) reacting, by chemical depolymerization by glycolysis, the raw material from step a) obtaining a mixture of the glycolysis product based on the intermediate bis-2-hydroxyethyl terephthalate (BHET) and low-molecular-weight small oligomers;
c) purifying the glycolysis product from step b) by using ion exchange resins and adsorbents for the removal of contaminants and colour, obtaining pure BHET monomer.

2. The method according to claim 1, comprising or consisting of steps a) to c) and additionally of the step d), of reacting, by polycondensation, the pure BHET monomer from step c), obtaining polyethylene terephthalate, and subsequently crystallizing the produced polyethylene terephthalate in a solid phase.

3. The method according to claim 1 or 2, wherein the raw material of polyethylene terephthalate (PET) can include or be comprised of post-consumer light, coloured, "light blue", opaque bottles, post-consumer multilayer items with layers of PET coupled with barrier polymers for gas or metallic or polyolefins, printed PET layers, PET fibres or films, such as fabrics or polyester terephthalates, such as poly-trimethylene terephthalate, or polybutylene terephthalate, of any form and type available from the collection of wastes or from industrial production wastes.

4. The method according to any one of claims 1 to 3, wherein **step a)** includes or consists of the following step:
a1) mechanical-physical pre-treatment of the incoming material through a series of steps of drying, separation of materials of other chemical composition, separation of metal content and reduction in scales giving a plastic material flake.

5. The method according to any one of claims 1 to 4, wherein **step b)** includes or consists of the following steps:
b1) preparing a paste composed of the mixture of mono-ethylene glycol and flakes coming from the pre-treatment step a) for the depolymerization reaction;
b2) glycolysis reaction in the presence of an excess mono-ethylene glycol to give bis-2-hydroxyethyl terephthalate (BHET) and small amounts of low molecular weight oligomers.

6. The method according to claim 5, wherein step b1) is carried out under the following conditions:
- molar ratios MEG:PET comprised between 8:1 and 2:1, preferably between 3:1 and 4:1;
- temperature comprised between 100°C and 200°C, preferably between 140°C and 160°C; and/or step b2) is carried out in the presence of a glycolysis catalyst, preferably selected from sodium carbonate, zinc chloride or zinc acetate, under the following conditions:
- molar ratios MEG:PET comprised between 8:1 and 2:1, preferably between 3:1 and 4:1;
- catalyst percentage comprised between 0.2-1.5%, preferably about 0.5-1% by weight;
- temperature comprised between 160°C and 260°C, preferably between 180°C and 250°C;
- reaction time comprised between 2 and 8 h, preferably between 4 and 8 h,
and wherein the method optionally includes a mechanical filtration step of the product from step b2) .

7. The method according to any one of claims 1 to 6, wherein **step c)** includes or consists of the following steps:
c1) purification and decolourization of the glycolysis product with cation exchange resins, anion exchange resins and, if necessary, decolorizing adsorbents, in order to remove chemical type impurities, such as metal cations, anions and pigments or colourings and to obtain the purified, clear, and almost colourless BHET monomer;
c2) recovery of the pigments or colourings discarded during the decolourization step by washing the resins from step c1) with a solvent and subsequent distillation of the solvent;
c3) purification of the glycolysis product by evaporation and concentration suitable to eliminate the excess glycol in order to aid the subsequent polycondensation process.

8. The method according to claim 7, wherein **step c1)** is carried out under the following conditions:
- glycolysis product/resin ratio comprised between 10:1 by volume and 1:1 by volume, preferably between 2:1 by volume and 1:1 by volume;
- temperature comprised between 50°C and 180°C, preferably between 70°C and 120°C;
- residence time comprised between 1 and 4 h, preferably between 1 and 2 h; and/or
**step c2)** is carried out under the following conditions:
- resin/solvent ratio comprised between 1:10 and 1:2, preferably between 1:5 and 1:7 by volume;
- washing time comprised between 1 and 4 h, preferably between 1 and 2 h;
- distillation temperature comprised between 40°C and 80°C, preferably between 40°C and 60°C;
- pressure comprised between 1000 and 100 mBar, preferably between 1000 and 500 mBar;
- distillation time comprised between 10 minutes and 1 h, preferably between 10 and 30 minutes; and/or
**step c3)** is carried out under the following conditions:
- temperature comprised between 100°C and 250°C, preferably between 110°C and 140°C;
- pressure comprised between 25 and 500 mBar, preferably between 25 and 200 mBar;
residence time comprised between 1 h and 9 h, preferably between 3 h and 8 h,
and, optionally, wherein the pure BHET obtained is stored in the molten state at a temperature comprised between 120°C and 180°C.

9. The method according to any one of claims 2 to 7, wherein **step d)** includes or consists of the following steps:
d1) polycondensation polymerization reaction of the BHET from step c3) under vacuum in the presence of a Lewis acid catalyst and one acid stabilizer to obtain polyethylene terephthalate (PET), wherein said step d1) includes the recovery of ethylene glycol via distillation;
d2) pelletization of PET in amorphous phase and solid phase crystallization of amorphous PET to obtain a crystalline pellet.

10. The method according to claim 9, wherein step d1) is carried out in the presence of a catalyst in a weight percentage of about 0.025%-0.035%, or about 0.030% and of the stabilizer in a weight percentage comprised between 0.0025% and 0.0035%, or about 0.0030%, wherein the catalyst is preferably antimony oxide (Sb₂O₃), or it is titanium oxide (TiO₂), while the stabilizer is preferably phosphoric acid (H₃PO₄).

11. The method according to claim 9 or 10, wherein step d1) is carried out by distillation of ethylene glycol under the following conditions:
- temperature between 250°C and 295°C, preferably between 280°C and 295°C;
- pressure between residual 30 and 1 mBar, preferably about residual 0.00133 Bar (= 1 mmHg);
- reaction time comprised between about 1 and 9 h, preferably between 2 and 4 h,
and wherein the reaction is deemed concluded at a degree of intrinsic viscosity (IV) of 0.60-0.74 (measured according to the ISO 1628-5 or ASTM D 4603 standard) and a weight average molecular weight of about 20000-30000.

12. The method according to any one of claims 9 to 11, wherein in **step d2),** the pelletization of amorphous PET is achieved by a submerged cutting system and wherein the crystallization reaction is carried out under a nitrogen flow to take the amorphous PET above its glass transition (Tg) temperature to a first temperature comprised between 190°C and 240°C, and subsequently to a second temperature comprised between 170°C and 220°C and for a total reaction time comprised between 12 and 48 h, preferably between 24 and 36 h, and wherein the reaction is deemed concluded at a weight average molecular weight >33000 with a consequent intrinsic viscosity value between 0,7 and 0,9 (measured according to the ISO 1628-5 standard) and a crystallinity percentage higher than or equal to 40%.

13. A pilot industrial plant for studying and scaling-up of the process according to any one of claims 1 to 12, including or consisting of the following operational units:
i) two or three stirred reactors and one oscillating tubular reactor (OPFR) configured for the glycolysis step b) two distillation and condensation systems, in which one consists of a distillation column, condenser and collector, the other consists only of a condenser and collector;
ii) a system of columns for the purification and decolorization by ion exchange resins and decolorizing adsorbents;
iii) a water pelletization system configured for the granulation of the molten material exiting the reactors and a double screw extruder for mixing and compounding operations on the products.

14. The pilot plant according to claim 13, wherein the **operational unit i** is consisting of the following equipment:
i1) a first stirred reactor (CSTR A) and a second stirred reactor (CSTR B), wherein said reactors (CSTR A, CSTR B) are preferably provided with a stirrer for highly viscous liquids and are preferably configured for the following maximum working temperature and pressures: shell 300°C/14 bar, jacket 350°C/10 bar;
i2) an oscillating tubular reactor (OPFR), preferably configured for the following maximum working temperature and pressures: shell 300°C/14 bar, jacket 350°C/10 bar;
i3) a distillation column (CDI) configured for separating water from ethylene glycol, the column (CDI) preferably having the following maximum working temperatures: in operation 190°C; designed 210°C, and the following maximum working pressures: between -1/1 bar; internal maximum pressure 3.5 bar; and/or
**the operational unit ii** includes or consists of the following equipment:
ii1) a mechanical filter (M) with 100 Mesh filtering mesh to retain impurities undissolved in the reaction step;
ii2) a system of heat exchangers (S) for cooling the glycolysis product exiting the reactor and entering the equipment ii3);
ii3) a plurality of columns placed in a series consisting of a first cationic resins (CC) column, a second anionic resins (CA) column and a third column filled with decolourizer (CD), for the removal of metals, anions and colour, respectively, by a resin system; and/or
**the operational unit iii** includes or is comprised of the following equipment:
iii1) double screw extruder (E);
iii2) tank and cutter (T) for the granulation of the molten material usable downhill from the extruder or in an independent manner directly exiting the stirred reactors for the granulation of the molten product,
wherein:
- **equipment i1-i3** is configured in such a way that, in a first operating condition, **steps b1), b2), c3)** can be carried out, and in a second operating condition **step d1)** can be carried out,
- **equipment ii1-ii3** is configured in such a way that the purification and decolorization of **steps c1) and c2)** can be carried out,
- **equipment iii1-iii2** is configured in such a way that the pelletization step of **step d2)** can be carried out.

15. An industrial plant for carrying out the method according to any one of claims 1 to 12, the plant including or consisting of the following operational units:
A) a series pre-treatment plant of a waste plastic material of polyester terephthalate, configured to obtain a flake of plastic material according to step a) suitable for a glycolysis treatment;
B) at least one loading reactor of the raw materials in flakes and one or more tubular oscillating reactors (OPFR) configured for the glycolysis reaction step according to step b);
C) a system of columns packed with ion exchange resins and adsorbents configured for the purification step c), said system of columns being preferably equipped with a regeneration system of the resins themselves and cooling of the incoming material;
D) a series of two CSTR reactors for the evaporation and concentration reactions and a stripping column for the recovery of the solvent;
E) optionally, one or more feeding tanks of the purified BHET intermediate obtained from the glycolysis step b);
F) a plurality of OPFR reactors divided in blocks for the polycondensation reaction of BHET according to step d);
G) a granulation system for the extrusion and production of pellets of polyethylene terephthalate and a finishing and crystallization system to obtain polyethylene terephthalate with the purity and the chemicalphysical and mechanical properties of virgin PET.

16. The industrial plant according to claim 15, wherein the **operational unit A** includes or consists of the following equipment:
A1) optionally, a metal chain conveyor configured to convey the polyester bales to the unbaling machine;
A2) optionally, an unbaling machine configured for manually cutting the steel packaging line of the packed bottles that will be then opened by a screw blade;
A3) a conveyor for conveying the material to the operational unit downhill, the conveyor being preferably a double screw loader configured for better separating the bottles;
A4) a roller screening system configured for separating impurities such as sand, rocks, metals, caps, based on the different sizes of the holes;
A5) an *eddy current* separator configured for separating non-magnetic metal fractions;
A6) a conveyor, preferably a belt one, configured for conveying material to the subsequent step;
A7) a label removal system configured for removing the labels by friction;
A8) a conveyor to convey the material to the operational unit downhill, the conveyor preferably consisting of a horizontal collection belt and a conveyor belt, configured to collect the bottles together from the label removal set and to transport the material to the subsequent step, respectively;
A9) a pre-washing apparatus for the removal of the labels in water, configured for removing labels and sand, earth and other impurities;
A10) a "metal detector" with "pneumatic turnover", configured for removing magnetic metals;
A11) a conveyor, preferably a belt one, configured for transporting material to the operational unit downhill;
A12) a milling system configured for cutting the material in small sizes;
A13) a conveyor configured for conveying the material flakes to the operational unit downhill, the conveyor preferably consisting of horizontal and oblique conveying screws;
A14) at least one floating basin, configured for gravity separating different materials from the minced polyester;
A15) a conveyor, preferably a loading screw, configured for conveying the materials out from the floating basin;
A16) a de-washing machine, configured for reducing the water content exiting the floating basin;
A17) a drying system, preferably a thermal tubing one, configured for reducing the humidity content and dry the material flakes by warm air;
A18) optionally, a zig-zag blower for label removal, configured for separating further small amounts of labels and dust from the material flakes;
A19) a second *eddy current* separator, configured for further separating non-magnetic metal fractions;
A20) a colour separator configured for separating flakes of different colours depending on the wavelength and for separating different types of plastics the ones from the others by infrared;
A21) optionally, one or more bins configured for temporary storing polyester flakes.

17. The industrial plant according to claim 15 or 16, wherein the **operational unit B** includes or consists of:
B1) at least one loading reactor, able to work under pressure and in vacuum, configured for preparing the glycolysis mixture;
B2) at least one OPFR reactor including a tubular reactor, configured for working under negative pressure conditions and equipped with a transfer pump; and/or
**the operational unit C** includes or consists of:
C1) a mechanical filter with a 1000-micron filtering mesh inside to retain impurities undissolved in the reaction step, resisting about 6 bar and 200°C;
C2) a cooling system for the incoming material in the C3) columns;
C3) a plurality of columns with a resin system for the removal of metals, anions and colour respectively, positioned in series and divided by type, preferably in three two-column groups with a maximum working temperature equal to 95°C, the columns C3) being preferably connected to a tubing system for the regeneration of the resins themselves, comprising a distillation column for pure acetone used in washing the resin decolourizer and consequent recovery of colour pigments as a precipitate; and/or
**operational unit D** includes or consists of:
D1) an evaporation reactor for the first evaporation step of the ethylene glycol solvent from the glycolysis product, configured for pressures up to 10 bar with vacuum system and temperature up to 350°C;
D2) a concentration reactor for the final elimination step of the ethylene glycol solvent and obtaining BHET with purity comprised between 80% and 99%, said reactor being preferably a CSTR reactor configured for pressures up to 10 bar with vacuum system and temperature up to 350°C;
D3) a stripping column for the condensation and recovery of pure ethylene glycol to be recycled in the process; and/or
**operational units F** include or consist of:
F1) a plurality of tubular OPFR reactors, preferably nine OPFR reactors, designed to work under negative pressure conditions and equipped with a transfer pump.

18. The industrial plant according to any one of claims 15 to 17, wherein the **operational unit G** includes or consists of:
G1) optionally, a deviating valve, usable to purge the cutting head from deteriorated material before starting the pelletization system;
G2) an immersion pelletization unit with a moveable support, configured in such a way that the molten polymer flows through a matrix having an array of holes, is cut in granules by rotating blades and is solidified in contact with a flow of process water flowing through the cutting chamber in the centrifugal dryer;
G3) a "Die Plate" unit to guarantee the thermal insulation that prevents freezing of the material at the matrix nozzle, warming cartridges;
G4) a cutting chamber configured for the transport of the cut pellets by the process water;
G5) optionally, a supporting frame with small wheels on which the whole pelletizer is mounted;
G6) a water and drying treatment system including a centrifugal dryer, to which the cut granules are conveyed via the process water pump and separated from the process water, so to reach a maximum residual pellet humidity of about 0.05-0.5%;
G7) optionally, a water tank configured for the required process water;
G8) optionally, a flow meter for the process water between the process water pump and the pelletization, displaying the actual flow rate in the control unit of the immersion pelletizer;
G9) a centrifugal dryer/desiccator;
G10) a discharge fan configured for extracting the water vapour, generating a counterflow to the flow of material from the granulate, to reach the target values of residual humidity;
G11) optionally, an electrical system with PLC wherein all the electric components required for control by the operator and monitoring of the machine are installed in the electric board;
G12) a solid-state warm crystallization system equipped with vibrating conveyor, that keeps the pellet initially still amorphous under continuous movement, in such way to prevent a conglutination of the pellets.
